(19) Europäisches Patentamt
European Patent Office
Office européen des brevets

(11) Numéro de publication: 0 255 452 B1

(12) # FASCICULE DE BREVET EUROPEEN

(45) Date de publication du fascicule du brevet: 24.01.90

(51) Int. Cl.[4]: C07C 37/62

(21) Numéro de dépôt: 87420183.3

(22) Date de dépôt: 01.07.87

(54) **Procédé de chloration de composés phénoliques.**

(30) Priorité: 02.07.86 FR 8609813

(43) Date de publication de la demande: 03.02.88 Bulletin 88/5

(45) Mention de la délivrance du brevet: 24.01.90 Bulletin 90/4

(84) Etats contractants désignés: AT BE CH DE ES GB GR IT LI LU NL SE

(56) Documents cités:
EP-A- 0 002 373
EP-A- 0 180 114
DE-A- 527 393
FR-A- 1 313 585

(73) Titulaire: RHONE-POULENC CHIMIE, 25, quai Paul Doumer, F-92408 Courbevoie Cédex(FR)

(72) Inventeur: Ratton, Serge, Hameau de Belmont Vaulx Milieu, F-38090 Villefontaine(FR)
Inventeur: Desmurs, Jean-Roger, La Jonquière Route de Ternay Communay, F-69360 St-Symphorien d'Ozon(FR)

(74) Mandataire: Vignally, Noel et al, RHONE-POULENC INTERSERVICES Service Brevets Chimie Centre de Recherches de Saint-Fons B.P. 62, F-69192 Saint-Fons Cedex(FR)

EP 0 255 452 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de chloration par le chlore gazeux de composés phénoliques substitués en positions ortho par rapport à la fonction hydroxyle.

Le brevet EP-A 0 002 373 décrit un procédé de préparation de trichloro-2,4,5 phénol par chloration du dichloro-2,5 phénol par le chlore, dans un milieu liquide inerte polaire et aprotique, et en présence d'un acide de Lewis lorsque le milieu réactionnel a une constante diélectrique inférieure à 30.

Le brevet EP-A 0 180 114 décrit un procédé de préparation de trichloro-2,4,5 phénol par chloration du dichloro-2,5 phénol à l'aide de chlorure de sulfuryle dans l'acide sulfurique concentré en présence d'un sulfure de diaryle comme catalyseur.

Le brevet FR 1 313 585 décrit la chloration de l'orthocrésol par le chlorure de sulfuryle, en présence d'un catalyseur halogénure de métal et de terre à foulon.

Le brevet DE-B 527 393 décrit la préparation de tétrachlorophénols et de pentachlorophénol, par chloration de phénol ou de chlorophénols allant jusqu'au stade de trichlorophénol, par le chlore, dans un solvant et en présence d'un agent chlorant tel que l'iode ou le chlorure de fer et à une température inférieure à 50°C.

Aucun de ces documents ne concerne la chloration de phénols substitués en positions 2 et 6.

Parmi les composés phénoliques importants, qui sont susceptibles d'être obtenus par chloration d'un composé phénolique substitué en positions ortho, se trouve le trichloro-2,4,6 phénol.

Le procédé habituel de préparation du trichloro-2,4,6 phénol consiste dans la chloration du dichloro-2,4 phénol.

Cependant il se forme une faible proportion de trichloro-2,4,5 phénol (de l'ordre de 0,003 à 0,0010% du poids de trichloro-2,4,6 phénol). Le trichloro-2,4,6 phénol, qui est un intermédiaire pour la synthèse d'autres composés, ne doit pas contenir de traces de cet isomère indésirable.

Une solution à ce problème consisterait donc à chlorer le dichloro-2,6 phénol, ce qui éviterait totalement la formation de trichloro-2,4,5 phénol. Le trichloro-2,3,6 phénol, susceptible de se former dans ce cas, à l'état de traces, est beaucoup moins gênant que le trichloro-2,4,5 phénol.

En fait, lorsque l'on chlore par le chlore gazeux le dichloro-2,6 phénol, on constate que l'on n'obtient pas un excellent rendement. Il se forme notamment une quantité importante de pentachloro-2,4,5,6,6 cyclohexène-2 one, ce qui rend le mélange réactionnel très instable et difficile à purifier.

La présente invention se propose de résoudre ce problème, ainsi que le problème plus général de la chloration en position para, avec un bon rendement, des composés phénoliques ayant des substituants en positions ortho par rapport à la fonction hydroxyle.

Plus précisément, elle concerne un procédé de chloration par le chlore gazeux de composés phénoliques de formule générale (I):

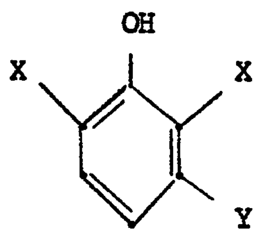

(I)

dans laquelle :

- les symboles X, identiques ou différents, représentent un atome de chlore, un atome de brome, un groupement méthyle ou éthyle, un groupement méthoxy ou éthoxy, un groupement acétoxy,
- le symbole Y représente un atome d'hydrogène, un groupement méthyle ou éthyle, un groupement méthoxy ou éthoxy,

caractérisé en ce que l'on opère à une température inférieure ou égale à 180°C en présence d'une quantité efficace d'un acide fort ou d'un acide de Lewis.

Dans le présent texte, on entend par acide fort un acide protonique ayant une fonction d'acidité Ho inférieure ou égale à -5.

Comme exemples non limitatifs de tels acides forts, on peut citer l'acide sulfurique, l'acide perchlorique, l'acide trifluorométhanesulfonique, l'acide chlorosulfonique, l'acide fluorosulfonique, l'acide pyrosulfurique et des résines acides comportant des groupements fluorosulfoniques.

Par acide de Lewis, on entend dans le présent texte, selon la définition usuelle, des composés accepteurs de doublets électroniques. On peut utiliser notamment les acides de Lewis cités dans l'ouvrage édité par G. A. OLAH "Friedel-Crafts and Related Reactions" tome I, pages 191 à 197 (1963).

Les acides de Lewis pouvant servir dans le procédé, sont plus particulièrement les halogénures d'éléments des groupes 3a, 4a, 5a, 1b, 2b, 4b, 5b, 6b, 7b et 8 de la classification périodique des éléments, qui sont liquides ou solides dans les conditions opératoires, tels que les chlorures, bromures, fluorures et iodures d'aluminium, d'étain, de phosphore, d'antimoine, d'arsenic, de bismuth, de titane, de tantale, de

nobium, de zirconium, de vanadium, de tungstène, de molybdène, de fer, de cobalt, de nickel, de cuivre, de zinc et de cadmium.

Comme exemples spécifiques de tels halogènures, on peut citer le chlorure d'aluminium, le bromure d'aluminium, les chlorures stannique et stanneux, les bromures stannique et stanneux, le trichlorure de bismuth, le tétrachlorure de titane, le tétrachlorure de zirconium, le pentafluorure d'antimoine, l'hexachlorure de tungstène, les chlorures de molybdène, le chlorure ferrique, le chlorure ferreux, le bromure ferrique, le bromure ferreux, le chlorure cuivreux, le chlorure cuivrique et le chlorure de zinc.

Parmi ces acides de Lewis, on préfère le chlorure d'aluminium, le chlorure ferrique, le tétrachlorure de zirconium et le tétrachlorure de titane.

On peut également utiliser des complexes de certains acides de Lewis avec un hydracide, dans la mesure où ces complexes sont liquides ou solides dans les conditions de la réaction. Ainsi on peut citer par exemple le complexe $SbF_5$, HF.

Généralement la quantité d'acide fort ou d'acide de Lewis utilisée est telle que le rapport pondéral acide fort/composé phénolique de formule (I) ou acide de Lewis/composé phénolique de formule (I) est de 0,01 % à 10 %.

De préférence ces rapports pondéraux sont de 0,1 % à 5 %.

Le procédé selon l'invention peut être conduit en l'absence de solvant, c'est-à-dire que les réactifs sont à l'état fondu.

A l'état fondu, on préfère plus particulièrement utiliser un acide protonique fort tel que défini précédemment.

Ainsi par exemple, on obtient avec un excellent rendement, du trichloro-2,4,6 phénol contenant généralement de 3% en poids de pentachloro-2,4,5,6,6 cyclohexène-2 one.

On peut également opérer dans un milieu liquide constitué par un acide carboxylique tel que l'acide acétique, un acide chloroacétique, un acide fluoroacétique ou l'acide propionique.

Lorsque l'on utilise un milieu liquide pour réaliser le procédé, il s'agit le plus souvent de l'acide acétique ou de l'acide trifluoroacétique.

En milieu liquide, on obtient de très bons résultats aussi bien avec un acide protonique fort qu'avec un acide de Lewis, tels que définis précédemment.

Ainsi par exemple, dans le cas de la chloration du dichloro-2,6 phénol, on a d'excellents rendements en trichloro-2,4,6 phénol, contenant généralement moins de 3 % en poids de pentachloro-2,4,5,6,6 cyclohexène-2 one.

La quantité de chlore utilisée dans le procédé selon l'invention est essentiellement fonction du taux de transformation souhaité du composé phénolique (I).

En pratique, le plus souvent, le chlore est introduit par barbotage dans le mélange réactionnel. La pression dans l'appareillage est donc sensiblement égale ou légèrement supérieure à la pression atmosphérique.

Le chlore peut être utilisé seul ou être dilué par un gaz inerte, tel que l'azote par exemple. La présence d'un gaz inerte permet, si nécessaire, d'augmenter le débit gazeux sans augmenter corrélativement la quantité de chlore introduite en un temps donné.

Le chlore gazeux utilisé dans le présent procédé peut également être formé in situ, à partir d'acide chlorhydrique, par addition d'un composé oxydant, tel que par exemple le peroxyde d'hydrogène.

La température à laquelle est mis en oeuvre le procédé de l'invention est généralement inférieure ou égale à 180°C. La limite inférieure n'est pas critique. Elle est conditionnée par la nécessité d'avoir un mélange réactionnel liquide.

Lorsque l'on opère à l'état fondu, cette température inférieure variera donc selon le composé phénolique (I) soumis à la chloration. Ainsi lorsque l'on chlore le dichloro-2,6 phénol, il faudra une température d'au moins 65°C.

Lorsque l'on opère en milieu acide carboxylique, on pourra descendre jusqu'à 20°C par exemple.

De préférence cependant la température sera comprise entre 40°C et 120°C, si l'on est en milieu acide carboxylique.

Si l'on opère à l'état fondu, les températures préférées seront également comprises entre 40°C et 120°C, sauf bien entendu pour les composés phénoliques ayant un point de fusion supérieur à 40°C, pour lesquels la zone préférée de température sera comprise entre leur point de fusion et 120°C.

Parmi les composés phénoliques de formule (I) auxquels peut s'appliquer le procédé de l'invention, on peut citer plus particulièrement le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol.

On peut, si on le souhaite, chlorer des mélanges de ces composés phénoliques.

Comme cela a été indiqué précédemment, le procédé de l'invention est tout particulièrement adapté à la chloration du dichloro-2,6 phénol en trichloro-2,4,6 phénol, car il permet d'obtenir ce dernier composé en limitant très fortement la formation de produits secondaires indésirables tels que la pentachloro-2,4,5,6,6 cyclohexène-2 one.

Les exemples qui suivent illustrent la présente invention.

EXEMPLE 1

Dans un réacteur en verre de 100 cm3, équipé d'un agitateur, d'une tubulure permettant l'arrivée du chlore gazeux, d'un thermomètre et surmonté d'un réfrigérant, on introduit les charges suivantes :

| | |
|---|---|
| – dichloro-2,6 phénol: | 32,6 g (0,2 mole) |
| – acide trifluorométhane-sulfonique: | 0,16 g ($1,07 \times 10^{-3}$ mole) |

On élève la température du mélange réactionnel à 70°C et, sous agitation, on commence à introduire le chlore gazeux avec un débit de 5 litres/heure.

La durée de chloration est de 54 minutes, ce qui correspond à 4,48 litres de chlore introduit (0,2 mole).

En fin de réaction, on purge l'ensemble de l'installation par un courant d'azote.

La masse réactionnelle finale est incolore à l'état fondu ; elle est blanc/jaune très pâle à l'état solide et pèse 38,42 g.

On analyse la masse réactionnelle par chromatographie en phase gazeuse (CPG) et par chromatographie en phase liquide haute pression (CPLHP).

On obtient les résultats suivants :

| | |
|---|---|
| – Taux de transformation (TT) du dichloro-2,6 phénol: | 83,5% |
| – Rendement (RT) en trichloro-2,4,6 phénol par rapport au dichloro-2,6 phénol transformé: | 99,8% |

On note d'autre part :
- l'absence de pentachlorocyclohexènone
- la présence d'une faible quantité de polychlorophénoxyphénol : RT = 0,07 %.

EXEMPLE 2

On opère comme dans l'exemple 1.

On charge :

| | |
|---|---|
| – dichloro-2,6 phénol: | 65,2 g (0,4 mole) |
| – acide trifluorométhane-sulfonique: | 0,33 g ($2,15 \times 10^{-3}$ mole) |

On effectue la chloration à 70°C avec un débit de chlore de 5 litres/heure ; afin de transformer la quasi totalité du dichloro-2,6 phénol, la quantité de chlore introduite est de 0,5 mole de chlore.

La masse réactionnelle obtenue est très légèrement jaune à l'état fondu et pratiquement blanche à l'état solide.

Elle a un poids de 79,50 g.

On obtient les résultats suivants :

| | |
|---|---|
| – TT du dichloro-2,6 phénol: | 99,8% |
| – RT en trichloro-2,4,6 phénol: | 96,0% |
| – RT en tétrachloro-2,3,4,6 phénol: | 1,5% |

On constate d'autre part :
- l'absence de pentachlorocyclohexènone,
- l'absence de polychlorophénoxyphénols.

EXEMPLE 3

On opère comme dans l'exemple 1.

On charge :

| | |
|---|---|
| – dichloro-2,6 phénol: | 32,6 g (0,2 mole) |
| – acide sulfurique à 95%: | 0,33 g |

On effectue la chloration à 70°C avec un débit de chlore de 5 litres/heure ; la quantité de chlore introduite est de 4,48 litres (0,2 mole).

On obtient les résultats suivants :

| | |
|---|---|
| – TT du dichloro-2,6 phénol: | 77,6% |
| – RT en trichloro-2,4,6 phénol: | 97,0% |
| – RT en tétrachloro-2,3,4,6 phénol: | 0,3% |
| – RT en pentachlorocyclohexènone: | 2,4% |

EXEMPLE 4

On opère comme dans l'exemple 1.

On charge :

| | |
|---|---|
| – dichloro-2,6 phénol: | 32,6 g (0,2 mole) |
| – acide perchlorique à 70%: | 0,33 g |

On effectue la chloration à 70°C avec un débit de chlore de 5 litres/heure ; la quantité de chlore introduite est de 4,48 litres (0,2 mole).

On obtient les résultats suivants :

| | |
|---|---|
| – TT du dichloro-2,6 phénol: | 84,8% |
| – RT en trichloro-2,4,6 phénol: | 99,0% |
| – RT en tétrachloro-2,3,4,6 phénol: | 0,3% |
| – RT en pentachlorocyclohexènone: | 1,0% |

EXEMPLE 5

Cet exemple illustre la chloration du dichloro-2,6 phénol en trichloro-2,4,6 phénol en milieu acide acétique.

On opère comme dans l'exemple 1, mais en utilisant un réacteur en verre de 250 cm3.

On charge :

| | |
|---|---|
| – dichloro-2,6 phénol: | 32,6 g (0,2 mole) |
| – acide acétique: | 100 $cm^3$ |
| – acide trifluorométhanesulfonique: | 0,16 g |

On effectue la chloration à 70°C avec un débit de chlore de 5 litres/heure ; la quantité de chlore introduite est de 4,48 litres (0,2 mole).

On obtient les résultats suivants :

| | |
|---|---|
| – TT du dichloro-2,6 phénol: | 90,0% |
| – RT en trichloro-2,4,6 phénol: | 99,8% |

On ne décèle pas de traces de pentachlorocyclohexènone et de polychlorophénoxyphénols.

EXEMPLES 6 à 8

On opère comme dans l'exemple 1. On charge :

| | |
|---|---|
| – dichloro-2,6 phénol: | 32,6 g (0,2 mole) |
| – acide protonique fort: | voir tableau (I) |
| – chlore (débit 5 litres/heure): | 0,2 mole |

Température de la réaction : 70°C
Durée de la chloration : 54 minutes.

Le tableau (I) ci-après indique la nature et la quantité du catalyseur utilisé ainsi que le TT du dichloro-2,6 phénol (DCP) et les RT en trichloro-2,4,6 phénol (TCP) et éventuellement en pentachlorocyclohexènone (PCCH), en polychlorophénoxyphénol (PCPP) et en tétrachloro-2,3,4,6 phénol (TTCP).

EXEMPLES 9 et 10

On opère comme dans l'exemple 5. On charge :

| | |
|---|---|
| – dichloro-2,6 phénol: | 32,6 g (0,2 mole) |
| – acide de Lewis: | voir tableau (II) |
| – acide acétique: | 100 $cm^3$ |
| – chlore (débit 5 litres/heure): | 0,2 mole |

Température de la réaction : 70°C
Durée de la chloration : 54 minutes.

Le tableau (II) ci-après indique la nature et la quantité du catalyseur utilisé ainsi que le TT du dichloro-2,6 phénol (DCP) et les RT en trichloro-2,4,6 phénol (TCP) et éventuellement en pentachlorocyclohexènone (PCCH), en polychlorophénoxyphénol (PCPP) et en tétrachloro-2,3,4,6 phénol (TTCP).

EXEMPLES 11 et 12

On opère comme dans l'exemple 1. On charge :

| | |
|---|---|
| – dichloro-2,6 phénol: | 32,6 g (0,2 mole) |
| – acide de Lewis: | voir tableau (III) |
| – chlore (débit 5 litres/heure): | 0,2 mole |

Température de la réaction : 70°C
Durée de la chloration : 54 minutes.

Le tableau (III) ci-après indique la nature et la quantité du catalyseur utilisé ainsi que le TT du dichloro-2,6 phénol (DCP) et les RT en trichloro-2,4,6 phénol (TCP) et éventuellement en pentachlorocyclohexènone (PCCH), en polychlorophénoxyphénol (PCPP) et en tétrachloro-2,3,4,6 phénol (TTCP).

Tableau (I)

| Essais | Catalyseur Nature | Quantité | TT du DCP | RT en TCP | RT en PCCH | RT en PCPP | RT en TTCP |
|---|---|---|---|---|---|---|---|
| Témoin | néant | – | 66,1% | 76,2% | 16,9% | – | – |
| Exemple 6 | $H_2SO_4$ | 1,63 g | 73,3% | 99,0% | 0,5% | – | 0,5% |
| Exemple 7 | $CF_3SO_3H$ | 0,33 g | 81,6% | 98,8% | 0,4% | – | 0,5% |
| Exemple 8 | $H_2SO_4$ | 0,01 g | 62,5% | 86,2% | 11,2% | – | 1,3% |

Tableau (II)

| Essais | Catalyseur Nature | Quantité | TT du DCP | RT en TCP | RT en PCCH | RT en PCPP | RT en TTCP |
|---|---|---|---|---|---|---|---|
| Exemple 9 | $AlCl_3$ | 0,33 g | 90,8% | 99,5% | – | 0,4% | – |
| Exemple 10 | $FeCl_3$ | 0,33 g | 84,2% | 99,1% | – | – | 0,7% |

Tableau (III)

| Essais | Catalyseur Nature | Quantité | TT du DCP | RT en TCP | RT en PCCH | RT en PCPP | RT en TTCP |
|---|---|---|---|---|---|---|---|
| Exemple 11 | $AlCl_3$ | 0,33 g | 93,4% | 83,3% | – | 0,8% | 14,2% |
| Exemple 12 | $FeCl_3$ | 0,33 g | 89,4% | 80,3% | – | 0,5% | 17,8% |

## Revendications

1. Procédé de chloration par le chlore gazeux de composés phénoliques de formule générale (I) :

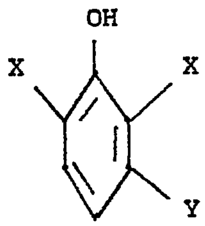

(I)

dans laquelle:

– les symboles X, identiques ou différents, représentent un atome de chlore, un atome de brome, un groupement méthyle ou éthyle, un groupement méthoxy ou éthoxy, un groupement acétoxy,

– le symbole Y représente un atome d'hydrogène, un groupement méthyle ou éthyle, un groupement méthoxy ou éthoxy,

caractérisé en ce que l'on opère à une température inférieure ou égale à 180°C, en présence d'une quantité efficace d'un acide fort protonique ayant une fonction d'acidité Ho inférieure ou égale à –5 ou d'un acide de Lewis.

2. Procédé selon la revendication 1, caractérisé en ce que l'acide fort est choisi dans le groupe comprenant l'acide sulfurique, l'acide perchlorique, l'acide trifluorométhanesulfonique, l'acide chlorosulfonique, l'acide fluorosulfonique, l'acide pyrosulfurique et des résines acides comportant des groupements fluorosulfoniques.

3. Procédé selon la revendication 1, caractérisé en ce que l'acide de Lewis est choisi parmi les chlorures, bromures, fluorures et iodures d'aluminium, d'étain, de phosphore, d'antimoine, d'arsenic, de bismuth, de titane, de tantale, de nobium, de zirconium, de vanadium, de tungstène, de molybdène, de fer, de cobalt, de nickel, de cuivre, de zinc et de cadmium et les complexes de certains acides de Lewis avec un hydracide.

4. Procédé selon l'une des revendications 1 ou 3, caractérisé en ce que l'acide de Lewis est choisi dans le groupe comprenant le chlorure d'aluminium, le bromure d'aluminium, les chlorures stannique et stanneux, les bromures stannique et stanneux, le trichlorure de bismuth, le tétrachlorure de titane, le tétrachlorure de zirconium, le pentafluorure d'antimoine, l'hexachlorure de tungstène, les chlorures de molybdène, le chlorure ferrique, le chlorure ferreux, le bromure ferrique, le bromure ferreux, le chlorure cuivreux, le chlorure cuivrique et le chlorure de zinc.

5. Procédé selon l'une des revendications 1, 3 ou 4, caractérisé en ce que l'acide de Lewis est choisi parmi le chlorure d'aluminium, le chlorure ferrique, le tétrachlorure de zirconium et le tétrachlorure de titane.

6. Procédé selon l'une des revendications 1 à 5, caractérisé en ce que le rapport pondéral acide fort/composés phénolique de formule (I) ou acide de Lewis/composé phénolique de formule (I) est de 0,01% à 10% et de préférence de 0,1% à 5%.

7. Procédé selon l'une des revendications 1 à 6, caractérisé en ce que les réactifs sont à l'état fondu.

8. Procédé selon l'une des revendications 1 à 6, caractérisé en ce que l'on opère en milieu liquide constitué par un acide carboxylique.

9. Procédé selon la revendication 8, caractérisé en ce que l'acide carboxylique est choisi parmi l'acide acétique, un acide chloroacétique, un acide fluoroacétique et l'acide propionique.

10. Procédé selon l'une des revendications 1 à 7, caractérisé en ce que l'on opère à une température comprise entre le point de fusion du ou des composés phénoliques de formule (I) et 180°C et de préférence entre le point de fusion du ou des composés phénoliques et 120°C.

11. Procédé selon l'une des revendications 1 à 6, 8 et 9, caractérisé en ce que l'on opère à une température comprise entre 20°C et 180°C, et de préférence comprise entre 40°C et 120°C.

12. Procédé selon l'une des revendications 1 à 11, caractérisé en ce que le composé phénolique (I) est le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol.

13. Procédé selon l'une des revendications 1 à 12, caractérisé en ce que le composé phénolique (I) est le dichloro-2,6 phénol.

**Patentansprüche**

1. Verfahren zur Chlorierung von phenolischen Verbindungen der allgemeinen Formel (I)

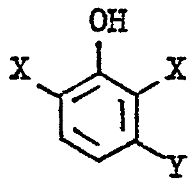

(I)

mit gasförmigem Chlor, worin die Symbole X, die identisch oder verschieden sind, ein Chloratom, ein Bromatom, eine Methyl- oder Ethylgruppe, eine Methoxy- oder Ethoxygruppe, eine Acetoxygruppe bedeuten, das Symbol Y ein Wasserstoffatom, eine Methyl- oder Ethylgruppe oder eine Methoxy- oder Ethoxygruppe bedeutet, dadurch gekennzeichnet, daß man bei einer Temperatur unterhalb oder gleich 180°C in Anwesenheit einer wirksamen Menge einer starken Protonsäure mit einer Säurefunktion Ho unterhalb oder gleich –5 oder einer Lewissäure arbeitet.

2. Verfahren gemäß Anspruch 1, dadurch gekennzeichnet, daß die starke Säure ausgewählt ist aus der Gruppe, bestehend aus Schwefelsäure, Perchlorsäure, Trifluormethansulfonsäure, Chlorsulfonsäure, Fluorsulfonsäure, Pyroschwefelsäure und den sauren Harzen mit Fluorsulfongruppen.

3. Verfahren gemäß Anspruch 1, dadurch gekennzeichnet, daß die Lewissäure ausgewählt ist unter den Chloriden, Bromiden, Fluoriden und Jodiden von Aluminium, Zinn, Phosphor, Antimon, Arsen, Wismut, Titan, Tantal, Niob, Zirkon, Vanadin, Wolfram, Molybdän, Eisen, Kobalt, Nickel, Kupfer, Zink und Cadmium und den Komplexen gewisser Lewissäuren mit einer Wasserstoffsäure.

4. Verfahren gemäß einem der Ansprüche 1 bis 3, dadurch gekennzeichnet, daß die Lewissäure ausgewählt ist aus der Gruppe, bestehend aus Aluminiumchlorid, Aluminiumbromid, den Stanni- und Stannochloriden, den Stanni- und Stannobromiden, Wismuttrichlorid, Titantetrachlorid, Zirkontetrachlorid, Antimonpentafluorid, Wolframhexachlorid, Molybdänchloriden, Ferrichlorid, Ferrochlorid, Ferribromid, Ferrobromid, Kuprochlorid, Kuprichlorid und Zinkchlorid.

5. Verfahren gemäß einem der Ansprüche 1, 3 oder 4, dadurch gekennzeichnet, daß die Lewissäure ausgewählt ist unter Aluminiumchlorid, Ferrichlorid, Zirkontetrachlorid und Titantetrachlorid.

6. Verfahren gemäß einem der Ansprüche 1 bis 5, dadurch gekennzeichnet, daß das Gewichtsverhältnis starke Säure/phenolische Verbindungen der Formel (I) oder Lewissäure/phenolische Verbindung der Formel (I) von 0,01% bis 10% und vorzugsweise von 0,1 bis 5% beträgt.

7. Verfahren gemäß einem der Ansprüche 1 bis 6, dadurch gekennzeichnet, daß die Reaktanten in geschmolzenem Zustand vorliegen.

8. Verfahren gemäß einem der Ansprüche 1 bis 6, dadurch gekennzeichnet, daß man in flüssigem Milieu, bestehend aus einer Carbonsäure, arbeitet.

9. Verfahren gemäß Anspruch 8, dadurch gekennzeichnet, daß die Carbonsäure ausgewählt ist unter Essigsäure, einer Chloressigsäure, einer Fluoressigsäure und Propionsäure.

10. Verfahren gemäß einem der Ansprüche 1 bis 7, dadurch gekennzeichnet, daß man bei einer Temperatur zwischen dem Schmelzpunkt des oder der phenolischen Verbindungen der Formel (I) und 180°C und vorzugsweise zwischen dem Schmelzpunkt der phenolischen Verbindung oder Verbindungen und 120°C arbeitet.

11. Verfahren gemäß einem der Ansprüche 1 bis 6, 8 und 9, dadurch gekennzeichnet, daß man bei einer Temperatur zwischen 20°C und 180°C und vorzugsweise zwischen 40°C und 120°C arbeitet.

12. Verfahren gemäß einem der Ansprüche 1 bis 11, dadurch gekennzeichnet, daß die phenolische Verbindung (I) 2,6-Dichlorphenol, 2,6-Dimethoxyphenol, 2-Chlor-6-methoxyphenol, 2-Chlor-6-methylphenol, 2,6-Dichlor-3-methylphenol, 2,6-Dichlor-3-methoxyphenol, 2-Brom-6-methoxyphenol ist.

13. Verfahren gemäß einem der Ansprüche 1 bis 12, dadurch gekennzeichnet, daß die phenolische Verbindung (I) 2,6-Dichlorphenol ist.

**Claims**

1. Process for chlorination with gaseous chlorine of phenolic compounds of general formula (I):

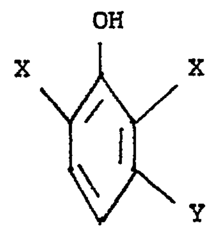

(I)

in which:
the symbols X, which are identical or different, denote a chlorine atom, a bromine atom, a methyl or ethyl group, a methoxy or ethoxy group or an acetoxy group, and
the symbol Y denotes a hydrogen atom, a methyl or ethyl group or a methoxy or ethoxy group, characterized in that it is conducted at a temperature lower than or equal to 180°C, in the presence of an effective quantity of a strong protonic acid which has an acidity function Ho lower than or equal to −5 or a Lewis acid.

2. Process according to claim 1, characterized in that the strong acid is chosen from the group comprising sulphuric acid, perchloric acid, trifluoromethanesulphonic acid, chlorosulphonic acid, fluorosulphonic acid, pyrosulphuric acid and acidic resins containing fluorosulphonic groups.

3. Process according to claim 1, characterized in that the Lewis acid is chosen from aluminium, tin, phosphorus, antimony, arsenic, bismuth, titanium, tantalum, niobium, zirconium, vanadium, tungsten, molybdenum, iron, cobalt, nickel, copper, zinc and cadmium chlorides, bromides, fluorides and iodides and the complexes of certain Lewis acids with a hydrogen acid.

4. Process according to one of claims 1 and 3, characterized in that the Lewis acid is chosen from the group comprising aluminium chloride, aluminium bromide, stannic and stannous chlorides, stannic and stannous bromides, bismuth trichloride, titanium tetrachloride, zirconium tetrachloride, antimony pentafluoride, tungsten hexachloride, molybdenum chlorides, ferric chloride, ferrous chloride, ferric bromide, ferrous bromide cuprous chloride, cupric chloride and zinc chloride.

5. Process according to one of claims 1, 3 and 4, characterized in that the Lewis acid is chosen from aluminium chloride, ferric chloride, zirconium tetrachloride and titanium tetrachloride.

6. Process according to one of claims 1 to 5, characterized in that the weight ratio of strong acid to the phenolic compound of formula (I) or of the Lewis acid to the phenolic compound of formula (I) is from 0.01% to 10% and preferably from 0.1% to 5%.

7. Process according to one of claims 1 to 6, characterized in that the reactants are in the molten state.

8. Process according to one of claims 1 to 6, characterized in that it is conducted in a liquid medium consisting of a carboxylic acid.

9. Process according to claim 8, characterized in that the carboxylic acid is chosen from acetic acid, a chloroacetic acid, a fluoroacetic acid and propionic acid.

10. Process according to one of claims 1 to 7, characterized in that it is conducted at a temperature between the melting point of the phenolic compound(s) of formula (I) and 180°C, and preferably between the melting point of the phenolic compound(s) and 120°C.

11. Process according to one of claims 1 to 6, 8 and 9, characterized in that it is conducted at a temperature between 20°C and 180°C, and preferably between 40°C and 120°C.

12. Process according to one of claims 1 to 11, characterized in that the phenolic compound (I) is 2,6-dichlorophenol, 2,6-dimethoxyphenol, 2-chloro-6-methoxyphenol, 2-chloro-6-methylphenol, 2,6-dichloro-3-methylphenol, 2,6-dichloro-3-methoxyphenol or 2-bromo-6-methoxyphenol.

13. Process according to one of claims 1 to 12, characterized in that the phenolic compound (I) is 2,6-dichlorophenol.